# EUROPEAN PATENT APPLICATION

(11) **EP 1 285 643 A2**
(43) Date of publication of application: **26.02.2003**
(21) Application number: 02255787.0
(22) Date of filing: 20.08.2002
(51) Int. Cl.: A61F 13/476, A61F 13/472

(54) **Absorbent article**

(30) Priority: 21.08.2001 JP 2001250084; 17.09.2001 JP 2001281905
(71) Applicant: UNI-CHARM CORPORATION, Kawanoe-shi Ehime-ken (JP)
(72) Inventor: Nozaki, Satoshi, c/o Technical Center, Mitoyo-gun, Kagawa-ken 769-1602 (JP); Yoshimasa, Wataru, c/o Technical Center, Mitoyo-gun, Kagawa-ken 769-1602 (JP)
(74) Representative: Parry, Christopher Stephen

(57) **Abstract**

Disclosed is an absorbent article including a main body portion and a pair of liquid impermeable members. Each liquid impermeable member includes front and rear end regions and an intermediate region therebetween. The intermediate region of each liquid impermeable member has a non-fixed portion to form a leakage preventing wall. At least one of the front and rear end regions of each liquid impermeable member has a flap which is allowed to be folded back, beyond corresponding one of transversely opposed side edges of the main body portion, against a garment surface of the main body portion.

## Description

### BACKGROUND OF THE INVENTION

### Field of the Invention

The present invention relates to an absorbent article suitable for use as sanitary napkin, urine absorbing pad, auxiliary absorbent pad for diaper and the like, more particularly, relates to an absorbent article in which the effect of preventing lateral leakage of liquid body exudates is enhanced by stabilizing the shape of leakage preventing walls.

### Description of the Related Art

There has been known an absorbent article having leakage preventing walls inside longitudinally-extending side edges so as to prevent lateral leakage of liquid body exudates such as menstrual blood or urine. In longitudinally opposed end regions, these leakage preventing walls are fixed on a surface of the absorbent article so as not to move away therefrom. In an intermediate region between the end regions, on the other hand, the leakage preventing walls are fixed only at their inwardly or outwardly facing side edges on the surface of the absorbent article so that their free ends (not-fixed side edges) can move away from the surface of the absorbent article. In the intermediate region, the leakage preventing walls are provided with elastic members for exhibiting an elastic contractive force.

Since the leakage preventing walls are fixed on the surface of the absorbent article in the end regions, a bending force due to the elastic contractive force of the elastic members longitudinally acts on the absorbent article, so that the absorbent article is curved. In the intermediate region, then, the leakage preventing walls are raised such that their free ends move away from the surface of the absorbent article. The leakage preventing walls thus raised come into contact with the skin of a wearer to dam up liquid body exudates trying to flow in a transverse (lateral) direction of the absorbent article, thereby enhancing the effect of preventing the lateral leakage of liquid body exudates.

In the conventional absorbent article, however, the leakage preventing walls are fixed in the end regions only on the surface of the absorbent article. Therefore, if the surface of the absorbent article is deformed during wear, the deformation of the surface directly affects the leakage preventing walls, so that the leakage preventing walls are easily irregularly deformed to follow the deformed shape of the surface.

In the case of sanitary napkin, for instance, after the sanitary napkin is attached to a crotch portion of an undergarment, the crotch portion is frequently strongly pulled up toward the crotch of the wearer's body for wearing the undergarment. Therefore, the absorbent article fixed on the crotch portion of the undergarment is easily convexly deformed to have its central portion protruded toward the wearer's crotch. At this time, in a sanitary napkin of the conventional structure, leakage preventing walls fixed only on the surface of the sanitary napkin are easily deformed to follow the convex deformation of the sanitary napkin, to thereby make their free ends irregularly fall down.

In the conventional absorbent article, moreover, the leakage preventing walls are integrated with the absorbent article over their entire length. Therefore, if the absorbent article is deformed during wear to reduce its width, for instance, the leakage preventing walls integrated with the absorbent article are also deformed to follow the deformation of the absorbent article, so that a relative position between the crotch portion of the undergarment and the leakage preventing walls is easily changed.

If the leakage preventing walls are irregularly deformed to follow the deformation of the absorbent article, as set forth above, the adhesion between the leakage preventing walls and the wearer's skin is decreased to cause a problem of lateral leakage of liquid body exudates beyond the leakage preventing walls or the like.

### SUMMARY OF THE INVENTION

The present invention has been worked out in view of the shortcoming in the prior art set forth above. It is therefore an object of the present invention to provide an absorbent article in which leakage preventing walls formed of liquid impermeable members are hardly irregularly deformed so that an effect of preventing lateral leakage due to the leakage preventing walls can be effectively exhibited.

According to the present invention, there is provided an absorbent article comprising:
a main body portion including a liquid permeable topsheet on a body surface of the main body portion, a backsheet on a garment surface of the main body portion and an absorbent layer disposed between the topsheet and the backsheet; and
a pair of liquid impermeable members spaced apart from each other transversely of the main body portion and extending longitudinally of the main body portion, each liquid impermeable member having longitudinally opposed front and rear end regions and an intermediate region between the front and rear end regions, wherein
the intermediate region of each liquid impermeable member has a longitudinally extending fixed portion, which is fixed on the body surface of the main body portion, and a non-fixed portion for forming a leakage preventing wall rising from the body surface of the main body portion, which is not fixed on the main body portion and on which an elastic contractive force acts so as to reduce a longitudinal dimension thereof, and
at least one of the front and rear end regions of each liquid impermeable member has a flap which is allowed to be folded back, beyond corresponding one of transversely opposed side edges of the main body portion, against the garment surface of the main body portion.

The flap may be folded back against the garment surface of the main body portion and fixed on the backsheet. In this case, since the flaps have been already fixed on the backsheet, wearers are not required to fold and fix the flaps on the backsheet or an undergarment upon use.

In an alternative, at least one of the flap and the backsheet may be provided with means for detachably fixing the flap on the backsheet when the flap is folded back against the garment surface of the main body portion. In this case, since the liquid impermeable members can be laid flat on the body surface of the main body portion until use, the absorbent article can be folded and individually packaged in a compact size suitable for potable use.

In another alternative, the flap may be provided with means for detachably fixing the flap on an outer side of an undergarment when the absorbent article is put on an inner side of the undergarment and the flap is folded back to come into contact with the outer side of the undergarment. In this case, too, since the liquid impermeable members can be laid flat on the body surface of the main body portion until use, the absorbent article can be folded and individually packaged in a compact size suitable for potable use.

In the case where the flaps are fixed on the backsheet, the flaps are constrained by the side edges of the main body portion. Therefore, even when the absorbent article is held between thighs and deformed to reduce its transverse dimension, the leakage preventing walls formed of the liquid impermeable members are hardly directly affected by the deformation of the body surface of the main body portion, thereby preventing irregular deformation of the leakage preventing walls. In the case where the flaps are fixed on the undergarment, on the other hand, the liquid impermeable members forming the leakage preventing walls are constrained by the undergarment. Therefore, even when a deforming force acts on the main body portion put on the inner side of the undergarment, a relative position between the crotch portion of the undergarment and the liquid impermeable members is hardly changed, so that the liquid impermeable members can be constantly kept in contact with preferred two side portions of a discharging part of the wearer's body. Of course, the irregular deformation of the leakage preventing walls can be prevented, as well.

In the present invention, it is possible that only one of the front and rear end regions of each liquid impermeable member is formed with the flap, and the other end region is fixed on the body surface of the main body portion as a whole so as not to be movable away from the body surface of the main body portion. In this case, since the leakage preventing walls hardly fall down outwardly, the position of the leakage preventing walls rising from the body surface of the main body portion can be certainly maintained.

In an alternative, both the front and rear end regions of each liquid impermeable member may be formed with flaps. In this case, the liquid impermeable member is hardly affected by the deformation of the main body portion. Especially when the individual flaps are fixed on the undergarment, a relative position between the undergarment and the liquid impermeable members are hardly changed.

In the present invention, it is also possible that the flap is not fixed on the body surface of the main body portion so as to be movable away from the body surface of the main body portion as a whole. In this case, the flap can be easily folded back, beyond the side edge of the undergarment, against the outer side of the undergarment.

In the present invention, it is also possible that the flap is extended outwardly beyond corresponding one of longitudinally opposed front and rear edges of the main body portion. With the liquid impermeable members thus extended, the shape of the leakage preventing walls can be stabilized when the flaps are fixed on the backsheet or undergarment.

Preferably, a boundary between the fixed portion and the non-fixed portion of the intermediate region is positioned above the absorbent layer. In this case, since the region having the absorbent layer provided therein is of a higher stiffness than the remaining region of the main body portion, the deformation of the leakage preventing wall rising therefrom can be made much difficult to cause.

Also preferably, a free edge of the liquid impermeable member is located closer to a longitudinally extending centerline of the main body portion than the fixed portion when the liquid impermeable member is not moved away from but remains laid on the body surface of the main body portion. In this case, the absorbent article can be folded in a compact size for individual packaging.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
Fig. 1 is a perspective view showing a sanitary napkin as an absorbent article according to a first embodiment of the present invention;
Fig. 2 is a top plan view showing a state where liquid impermeable members are laid flat on a body surface of a main body portion of the sanitary napkin of Fig. 1;
Fig. 3 is a top plan view showing a state where the liquid impermeable members are turned transversely outwardly of the main body portion of the sanitary napkin of Fig. 1;
Fig. 4 is a sectional view of the sanitary napkin taken along line IV - IV of Fig. 1;
Fig. 5 is a top plan view showing a sanitary napkin according to a second embodiment of the present invention;
Fig. 6 is a top plan view showing a state where liquid impermeable members of the sanitary napkin of Fig. 5 are turned;
Fig. 7 shows a state where the sanitary napkin of Fig. 5 is attached to a crotch portion of an undergarment, including a section of the sanitary napkin taken along line VII - VII of Fig. 6 together with a section of the undergarment;
Fig. 8 is a top plan view showing a sanitary napkin according to a third embodiment of the present invention;
Fig. 9 is a top plan view showing a sanitary napkin according to a fourth embodiment of the present invention; and
Fig. 10 is a top plan view showing a sanitary napkin according to a fifth embodiment of the present invention.

### DESCRIPTION OF THE PREFERRED EMBODIMENT

The present invention will be discussed hereinafter in detail in terms of the preferred embodiments according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

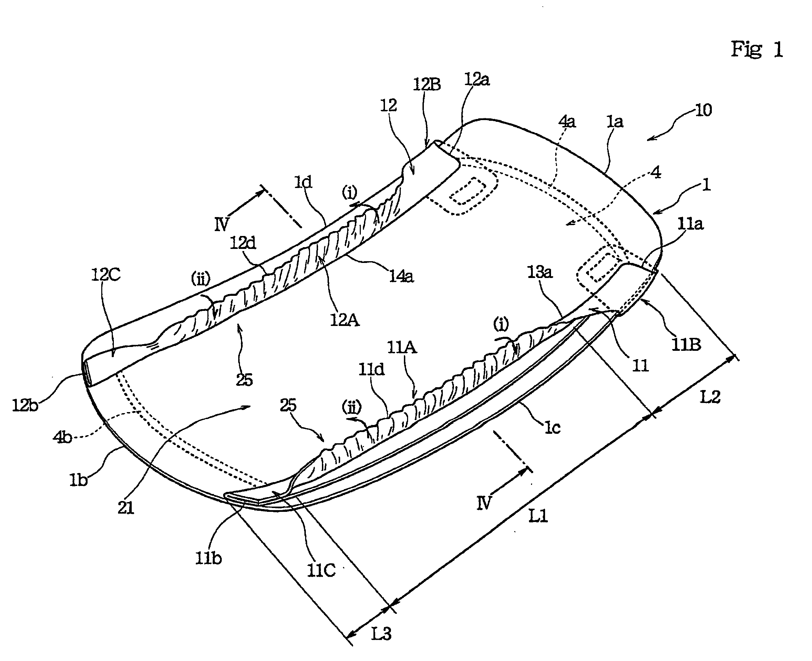

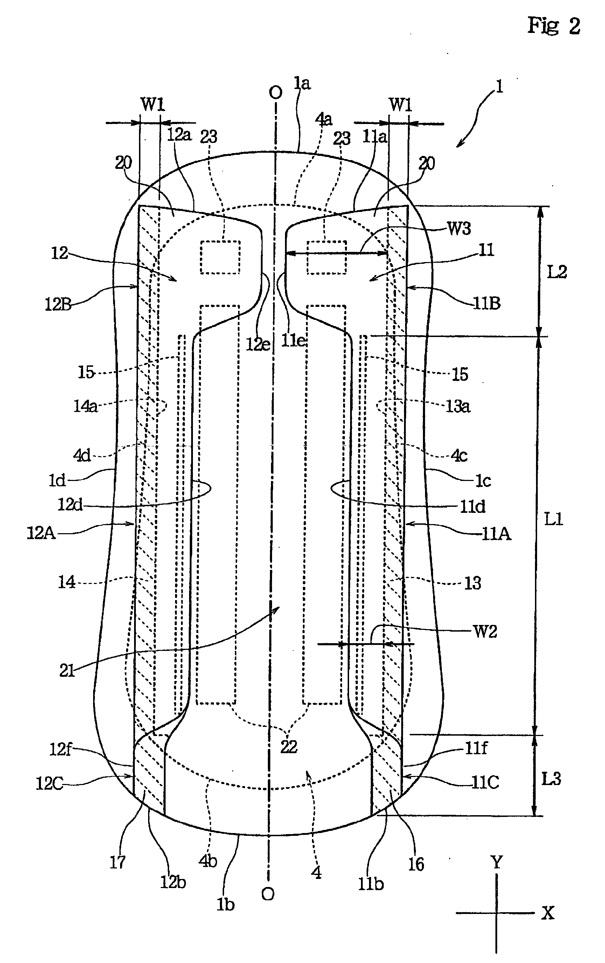

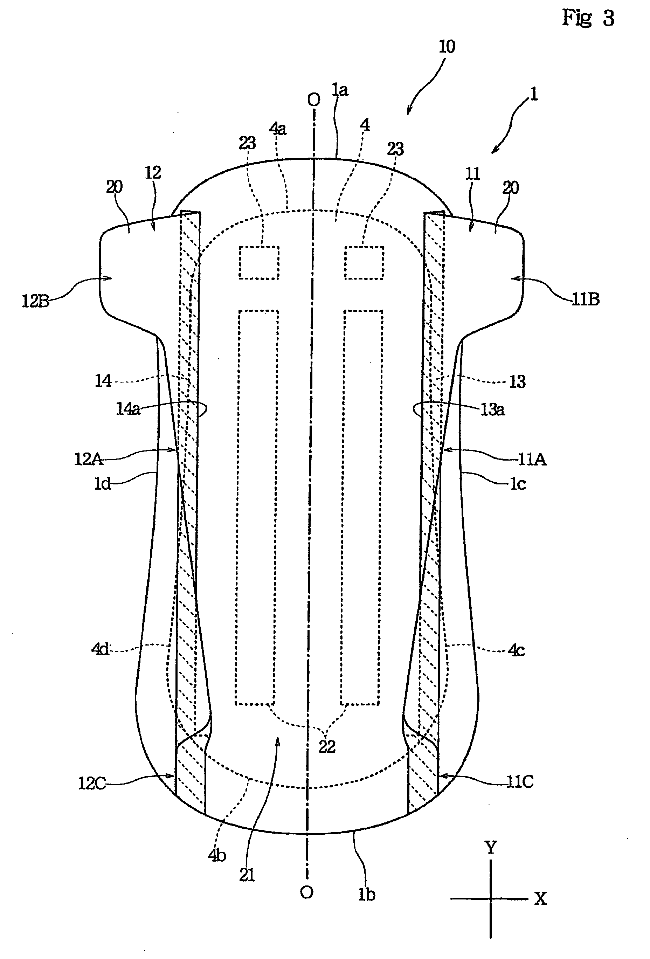

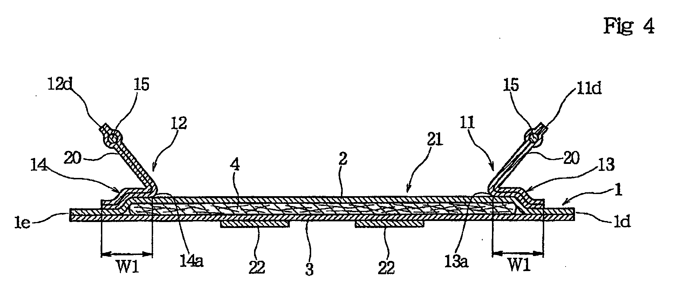

Fig. 1 is a perspective view showing a sanitary napkin 10 in a worn state; Fig. 2 is a top plan view showing how a main body portion and liquid impermeable members are initially joined; Fig. 3 is a top plan view showing a state where the liquid impermeable members are transversely outwardly opened; and Fig. 4 is a sectional view taken along line IV - IV of Fig. 1.

The sanitary napkin 10 shown in Figs. 1 to 4 is to be worn by a female during menstruation while being attached to an inner side of a crotch portion of an undergarment.

As shown in the top plan views of Figs. 2 and 3, the sanitary napkin has a main body portion 1 having an approximately arcuate front edge 1a, a rear edge 1b which is also arcuate, a right side edge 1c and a left side edge 1d, where the distance between the right side edge 1c and the left side edge 1d, i.e., the transverse dimension (width) of the main body portion 1 is slightly larger in the rear portion than in the front portion of the main body portion 1. On the other hand, the main body portion 1 has a body surface and a garment surface. As used herein, " body surface" means that surface which is intended to be worn toward or adjacent to the body of the wearer, while the " garment surface" is on the opposite side and is intended to be worn toward or placed adjacent to the undergarment when the sanitary napkin is worn.

As shown in Fig. 4, the main body portion 1 is of a layered structure comprising: a liquid permeable topsheet 2; a liquid impermeable backsheet 3; and an absorbent layer 4 disposed between the topsheet 2 and the backsheet 3 and having an ability to absorb a liquid and retain the absorbed liquid.

The topsheet 2 and the backsheet 3 are of the same shape and size, so that front edges, rear edges, right side edges and left side edges of the topsheet 2 and backsheet 3 coincide with the front edge 1a, rear edge 1b, right side edge 1c and left side edge 1d of the main body portion 1, respectively.

The absorbent layer 4 is of a given thickness, and has a front edge 4a, a rear edge 4b, a right side edge 4c and a left side edge 4d, as indicated by dotted line in Figs. 1 and 2. The front edge 4a and rear edge 4b of the absorbent layer 4 are similar in shape to the front edge 1a and rear edge 1b of the main body portion 1, respectively, and the right side edge 4c and left side edge 4d of the absorbent layer 4 are similar in shape to the right side edge 1c and left side edge 1d of the main body portion 1, respectively.

The front edge 4a, rear edge 4b, right side edge 4c and left side edge 4d of the absorbent layer 4 are inwardly spaced 3 to 10 mm apart from the front edge 1a, rear edge 1b, right side edge 1c and left side edge 1d of the main body portion 1, respectively. In a region outside the front edge 4a, rear edge 4b, right side edge 4c and left side edge 4d of the absorbent layer 4, the topsheet 2 and the backsheet 3 are bonded through a hot-melt adhesive or the like.

On the body surface of the main body portion 1, liquid impermeable members 11 and 12 are provided at positions transversely equally spaced apart from a longitudinally extending centerline O - O of the main body portion 1. These liquid impermeable members 11 and 12 are spaced apart from each other transversely (in X-direction) of the main body portion 1 and extended longitudinally (in Y-direction) of the main body portion 1 in a parallel relationship with each other.

Throughout this disclosure, each liquid impermeable member is longitudinally sectionalized (divided) into three regions having the same length (longitudinal dimension). In more detail, a region adjacent to the front edge of the main body portion and extending over 1/3 the length of the liquid impermeable member is referred to as " front end region" , a region adjacent to the rear edge of the main body portion and extending over 1/3 the length of the liquid impermeable member is referred to as " rear end region" , and a region between the front and rear end regions is referred to as " intermediate region" .

In addition, each liquid impermeable member is formed with at least one flap, as will be described later. The term " flap" as used herein means a portion which can protrude beyond corresponding one of the right and left side edges of the main body portion, when turned away from the longitudinally extending centerline of the main body portion, and which can be folded back, beyond the corresponding side edge, against the garment surface of the main body portion, and which has a sufficient size to fix it on the garment surface of the main body portion.

Here, at least one of the front and rear end regions of each liquid impermeable member is formed with such flap. The flap may extend over a part or the whole of the corresponding end region. If desired, the flap may extend from the corresponding end region to the intermediate region.

Here, it should be noted that, except for subsequent, additional manufacturing processes such as folding, individual packaging, etc., assembly of the sanitary napkin 10 of the first embodiment may be completed in either a state shown in Fig. 1 or a state shown in Fig. 2. For example, when the sanitary napkin is taken out of a packaging material by a wearer for use, the liquid impermeable members 11 and 12 may have been already partly folded back against the garment surface of the main body portion 1 and fixed thereon, or the liquid impermeable members 11 and 12 may remain laid on the body surface of the main body portion 1. In the latter case, thereafter, the liquid impermeable members 11 and 12 are transversely turned by the wearer herself from the state of Fig. 2 to the state of Fig. 3, and then, partly folded back against the garment surface of the main body portion 1 into the state of Fig. 1 for wearing.

As has been described above, the shape of the sanitary napkin is different between the former case and the latter case when the assembly is completed, but since the structure of the main body portion 1 and the shape of the liquid impermeable members 11 and 12 are substantially common to both the cases, the sanitary napkin 10 is used for describing both the cases.

The liquid impermeable member 11 comprises: a leakage preventing wall-forming portion 11A of a length L1; a flap 11B of a length L2, which includes a front edge 11a; and a rear end portion 11C of a length L3, which includes a rear edge 11b. The flap 11B extends over a part of the front end region; the rear end portion 11C extends over a part of the rear end region; and the leakage preventing wall-forming portion 11A extends over the whole of the intermediate region and the remaining parts of the front and rear end regions. Likewise, the liquid impermeable member 12 comprises: a leakage preventing wall-forming portion 12A of a length L1; a flap 12B of a length L2, which includes a front edge 12a; and a rear end portion 12C of a length L3, which includes a rear edge 12b. The flap 12B extends over a part of the front end region; the rear end portion 12C extends over a part of the rear end region; and the leakage preventing wall-forming portion 12A extends over the whole of the intermediate region and the remaining parts of the front and rear end regions.

As shown in Fig. 4, the individual liquid impermeable members 11 and 12 are formed of an elongated, strip-shaped liquid impermeable sheet 20, which is folded in two at a longitudinally extending fold lines and then trimmed. In the flap 11B, the liquid impermeable member 11 has a free edge 11e at the fold line. In the leakage preventing wall-forming portion 11A, on the other hand, the liquid impermeable member 11 has a free edge 11d along which the two-ply liquid impermeable sheet 20 is cut.

In the leakage preventing wall-forming portion 11A and the flap 11B, the two-ply liquid impermeable sheet 20 is fixed on the body surface of the main body portion 1 through an adhesive or by heat sealing to form a fixed portion 13 extending longitudinally of the main body portion 1 and having a width W1. In the rear end portion 11C, on the other hand, the liquid impermeable sheet 20 is further folded to orient an edge 11f, which is continued from the free edge 11d of the leakage preventing wall-forming portion 11A, to the right side edge 1c. In the rear end portion 11C, then, the liquid impermeable member 11 is fixed as a whole to form a fixed portion 16, so as not to move away from the body surface of the main body portion 1. This fixed portion 16 is also formed by adhesive bonding using an adhesive or heat sealing.

The liquid impermeable member 12 is of configuration symmetrical to the liquid impermeable member 11, and has a free edge 12e in the flap 12B and a free edge 12d in the leakage preventing wall-forming portion 12A. In the flap 12B and the leakage preventing wall-forming portion 12A, moreover, the liquid impermeable member 12 is fixed on the body surface of the main body portion 1 to form a longitudinally extending fixed portion 14 of a width W1. In the rear end portion 12C, on the other hand, the liquid impermeable sheet 20 is folded to orient an edge 12f, which is continued from the free edge 12d of the leakage preventing wall-forming portion 12A, to the left side edge 1d. In the rear end portion 12C, then, the liquid impermeable member 12 thus folded is fixed as a whole to form a fixed portion 17, so as not to move away from the body surface of the main body portion 1. This fixed portion 17 is formed by adhesive bonding or heat sealing.

As shown in Figs. 1 and 2, the fixed portion 13 is provided to extend over the total length of the leakage preventing wall-forming portion 11A and the flap 11B, and the fixed portion 14 is provided to extend over the total length of the leakage preventing wall-forming portion 12A and the flap 12B. In the leakage preventing wall-forming portion 11A and the flap 11B, therefore, the liquid impermeable member 11 has a free portion (non-fixed portion), which extends from a longitudinally extending starting edge 13a (which is the inner boundary of the fixed portion 13) to the free edges 11d and 11e and can be turned toward the right side edge 1c away from the body surface of the main body portion 1. In the leakage preventing wall-forming portion 12A and the flap 12B, similarly, the liquid impermeable member 12 has a free portion (non-fixed portion), which extends from a longitudinally extending starting edge 14a (which is the inner boundary of the fixed portion 14) to the free edges 12d and 12e and can be turned toward the left side edge 1d away from the body surface of the main body portion 1.

In the embodiment shown, the starting edges 13a and 14a are inwardly spaced apart from the right and left side edges 4c and 4d of the absorbent layer 4, respectively, to extend over the absorbent layer 4.

Over the entire length of the leakage preventing wall-forming portions 11A and 12A, the liquid impermeable members 11 and 12 are provided with elastic members 15 and 15 such that each elastic member 15 is disposed in and fixed to the two-ply liquid impermeable sheet 20. The individual elastic members 15 are joined to the liquid impermeable sheet 20 while being longitudinally stretched. As a result, in the leakage preventing wall-forming portions 11A and 12A, an elastic contractive force acts on the liquid impermeable members 11 and 12 to reduce their longitudinal dimension.

In the leakage preventing wall-forming portions 11A and 12A, as shown in Fig. 2, the free portions (non-fixed portions) of the liquid impermeable members 11 and 12 have a transverse dimension (width) W2. That is, the transverse dimension from the starting edges 13a, 14a to the free edges 11d, 12d is indicated by W2. In the flaps 11B and 12B, on the other hand, the free portions (non-fixed portions) of the liquid impermeable members 11 and 12 have a transverse dimension (width) W3. That is, the transverse dimension from the starting edges 13a, 14a to the free edges lie, 12e is indicated by W3. Here, the width W3 is set sufficiently larger than the width W2.

In the sanitary napkin 10, the region positioned between the transversely opposed starting edges 13a and 14a and having the absorbent layer 4 therein is a liquid-receiving region 21. On the garment surface of the main body portion 1, i.e., on an exterior surface of the backsheet 3, on the other hand, there are provided pressure sensitive adhesive layers 22, 22 as means for fixing the main body portion on the undergarment. The pressure sensitive adhesive layers 22, 22 are of hot-melt type, and applied to the exterior surface of the backsheet 3 in the shape of longitudinally extending strips.

In the sanitary napkin 10, the liquid impermeable members 11 and 12 of Fig. 2 can be transversely outwardly turned about the starting edges 13a and 14a, as shown in Fig. 3, and then, the flaps 11B and 12B of the liquid impermeable members 11 and 12 can be folded back, beyond the right and left side edges 1c and 1d of the main body portion 1, against the garment surface of the main body portion 1, as shown in Fig. 1.

In the case where the assembly of the sanitary napkin should be completed in the state of Fig. 1, the flaps 11B and 12B thus folded back are fixed on the garment surface of the main body portion 1 (i.e., the exterior surface of the backsheet 3). Fixing at this time is performed so as to prevent the flaps from being easily detached. For example, they are fixed by using a hot-melt type adhesive, heat sealing or stitching. In this case, subsequently, the sanitary napkin thus completed is folded in a compact form and then individually packaged in a packaging sheet.

In the case where the assembly of the sanitary napkin should be completed in the state of Fig. 2, pressure sensitive adhesive layers 23 and 23 as means for detachably fixing the flaps are provided on the exterior surface of the backsheet 3, as shown in Fig. 2. In an alternative, pressure sensitive adhesive layers may be provided on the surfaces of the flaps 11B and 12B, which appear in Fig. 2. In another alternative, the flaps 11B and 12B may be made detachable to the backsheet 3 by providing Hook-and-Loop fastener such that male sheets are disposed on the surfaces of the flaps 11B and 12B and female sheets are disposed on the exterior surface of the backsheet 3, or vice versa. In this case, the liquid impermeable members 11 and 12 are laid flat on the body surface of the main body portion 1 until use to partially cover the liquid-receiving region 21, as shown in Fig. 2. At this time, the free edges 11d and 12d of the leakage preventing wall-forming portions 11A and 12A and the free edges 11e and 12e of the flaps 11B and 12B are positioned closer to the centerline O - O than the fixed portions 13 and 14 and the starting edges 13a and 14a. Here, the flaps 11B and 12B may overlap. Subsequently, the sanitary napkin 10 thus completed is further folded in a compact form and then individually packaged in a packaging sheet for sale. After the individually packaged product is unwrapped by the wearer herself for use, the sanitary napkin 10 is unfolded to the state shown in Fig. 2. Subsequently, the liquid impermeable members 11 and 12 are transversely outwardly turned about the starting edges 13a and 14a to the state shown in Fig. 3 by the wearer herself, and then, the flaps 11B and 12B are folded back, beyond the right and left side edges 1c and 1d of the main body portion 1, against the garment surface of the main body portion 1. Finally, the flaps 11B and 12B are fixed on the exterior surface of the backsheet 3 of the main body portion 1 through the fixing means such as the pressure sensitive adhesive layers 23 and 23, so that the sanitary napkin is in the state of Fig. 1. The sanitary napkin 10 thus assembled to the state of Fig. 1 by the wearer herself is then worn.

When the sanitary napkin 10 is in the state of Fig. 1, a longitudinal elastic contractive force due to the elastic members 15 and 15 acts on the leakage preventing wall-forming portions 11A and 12A of the liquid impermeable members 11 and 12. With such elastic contractive force, the main body portion 1 is curved as shown in Fig. 1, and as a result, the leakage preventing wall-forming portions 11A and 12A are raised such that their free edges 11d and 12d move away from the body surface of the main body portion 1.

In the state of Fig. 2, the liquid impermeable members 11 and 12 are fixed, at their fixed portions 13 and 14, on the body surface of the main body portion 1, with their free edges 11d, 11e and 12d, 12e directed to the centerline O - O. In such state, therefore, the leakage preventing wall-forming portions 11A and 12A try to rise obliquely with their free edges 11d and 12d directed toward the centerline O - O. However, when worn, the flaps 11B and 12B (including the free edges 11e and 12e) are folded back, beyond the right and left side edges 1c and 1d, against the garment surface of the main body portion 1. In positions close to the flaps 11B and 12B, therefore, the free edges 11d and 12d of the leakage preventing wall-forming portions 11A and 12A are directed outwardly (indicated by arrows (i) in Fig. 1). In positions close to the rear end portions 11C and 12C, on the other hand, the free edges 11d and 12d of the leakage preventing wall-forming portions 11A and 12A are directed inwardly (indicated by arrows (ii) in Fig. 1).

Upon wearing, the sanitary napkin 10 in the state of Fig. 1 is fixed on an inner side of a crotch portion of an undergarment through the pressure sensitive adhesive layers 22 and 22 disposed on the backsheet 3, and then, worn together with the undergarment so as to bring the liquid-receiving region 21 of the main body portion 1 into contact with the crotch of the wearer's body.

Since the flaps 11B and 12B of the liquid impermeable members 11 and 12 are wrapped around the right and left side edges 1c and 1d of the main body portion 1 to be constrained by the right and left side edges 1c and 1d, even if the body surface of the main body portion 1 is deformed during wear, such deformation of the body surface hardly directly affects the shape of the leakage preventing wall-forming portions 11A and 12A, so that the leakage preventing walls can be certainly maintained in the position of Fig. 1 to have their front portions directed obliquely outwardly and their rear portions directed obliquely inwardly.

More specifically, when the sanitary napkin is worn by pulling up the undergarment, the main body portion 1 of the sanitary napkin fixed on the crotch portion of the undergarment tends to deform to reduce its transverse dimension, so that the body surface of the main body portion 1 is easily undulated. Even when the body surface of the main body portion 1 is thus undulated, since the flaps 11B and 12B are constrained by the right and left side edges 1c and 1d, the shape of the liquid impermeable members 11 and 12 hardly changes. Accordingly, during wear at the wearer's crotch, the leakage preventing walls can be maintained in the standing position of Fig. 1, without irregularly falling down or deforming. As a result, liquid body exudates trying to flow transversely out of the liquid-receiving region 21 of the main body portion 1 can be easily dammed up by the leakage preventing wall-forming portions 11A and 12A.

In addition, since the leakage preventing wall-forming portions 11A and 12A are inwardly inclined in their rear portions with their free edges 11d and 12d directed to the centerline O - O, as shown in Fig. 1, pocket-like portions 25 and 25 are formed between the leakage preventing wall-forming portions 11A and 12A and the body surface of the main body portion 1. Moreover, the shape of the pocket-like portions 25 and 25 can be certainly maintained without irregular deformation. Accordingly, liquid body exudates can be easily dammed up by the pocket-like portions 25 and 25, thereby preventing lateral leakage of the liquid body exudates. Furthermore, since the pocket-like portions 25 and 25 are provided in the rear portions, lateral leakage of liquid body exudates moving toward buttocks of the wearer's body can be easily prevented.

In the shown embodiment, moreover, the base ends of the leakage preventing wall-forming portions 11A and 12A are positioned above the absorbent layer 4. Since the region having the absorbent layer 4 is relatively stiff, a deformation force, which will be applied to the leakage preventing walls when the main body portion 1 is deformed, can be further decreased.

Here, the width W2 from the starting edge 13a, 14a to the free edge 11d, 12d in the leakage preventing wall-forming portions 11A and 12A is preferably equal to or more than 3 mm. If it is less than 3 mm, the liquid barrier effect due to the leakage preventing wall-forming portions 11A and 12A will be deteriorated. On the other hand, the upper limit of the width W2 is preferably 20 mm. If it is more than 20 mm, the leakage preventing wall-forming portions 11A and 12A will be easily folded or twisted when applied a pressure from the wearer's body.

On the other hand, the width W3 in the flaps 11B and 12B is preferable in a range of 15 to 40 mm. Within the above-mentioned range, the flaps can be easily folded back against the garment surface of the main body portion 1.

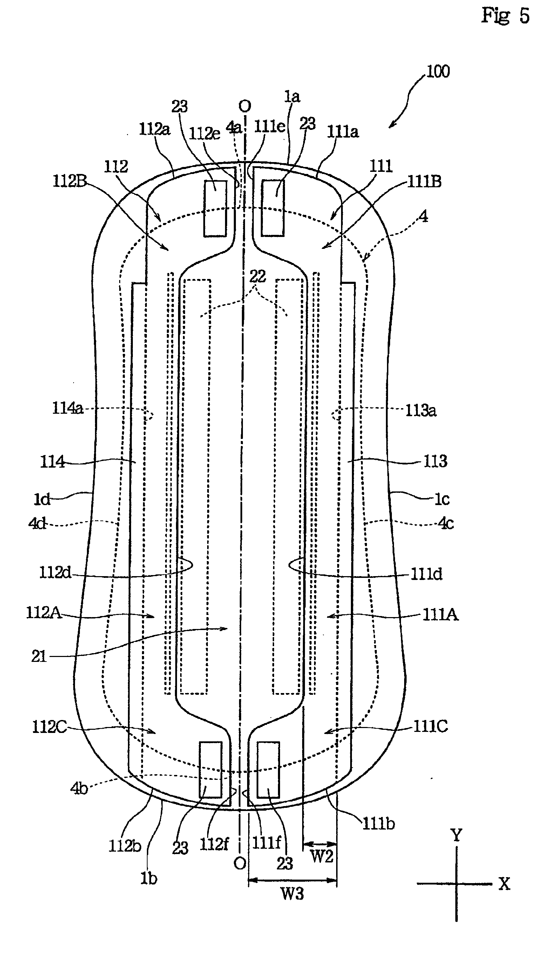

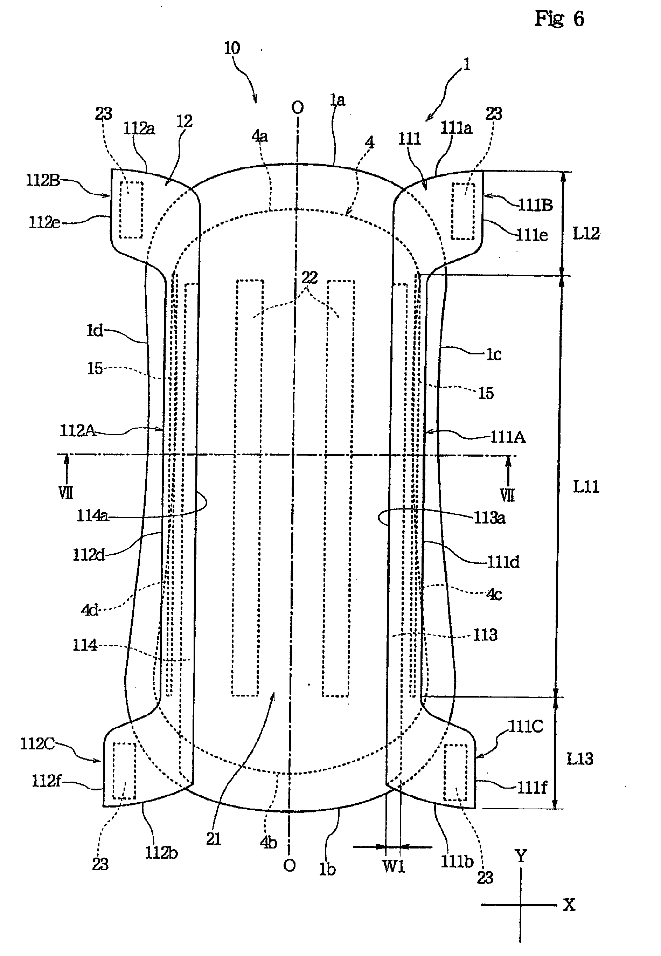

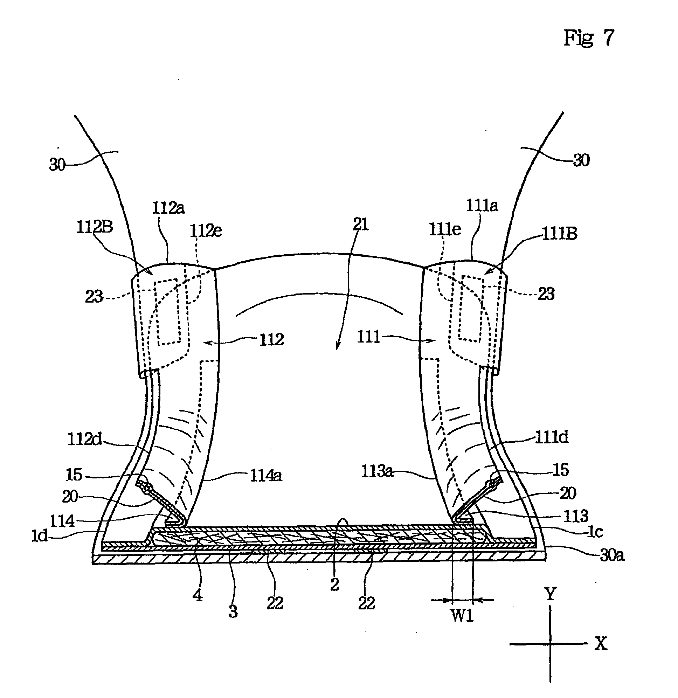

Fig. 5 is a top plan view showing a sanitary napkin 100 as an absorbent article according to a second embodiment of the present invention; Fig. 6 is a top plan view showing a state where liquid impermeable members are turned toward transversely opposed side edges of the sanitary napkin 100; and Fig. 7 shows a state where the sanitary napkin 100 is attached to a crotch portion of an undergarment, including a section of the sanitary napkin 100 taken along line VII - VII of Fig. 6 together with a section of the undergarment.

In the sanitary napkin 100 of the second embodiment, since the main body portion has the same shape and structure as those of the first embodiment shown in Figs. 1 to 4, the detailed description of the portions identical to those of Figs. 1 to 4 will be omitted by designating them by the common reference numerals.

In this second embodiment, too, liquid impermeable members 111 and 112 are provided at positions transversely equally spaced apart from a longitudinally extending centerline O - O of the main body portion 1, on the body surface of the main body portion 1. These liquid impermeable members 111 and 112 are spaced apart from each other transversely (in X-direction) of the main body portion 1 and extended longitudinally (in Y-direction) of the main body portion 1 in a parallel relationship with each other.

The liquid impermeable member 111 comprises: a leakage preventing wall-forming portion 111A of a length L11; a flap 111B of a length L12, which includes a front edge 111a; and a flap 111C of a length L13, which includes a rear edge 111b. The flap 111B extends over a part of the front end region; the flap 111C extends over a part of the rear end region; and the leakage preventing wall-forming portion 111A extends over the whole of the intermediate region and the remaining parts of the front and rear end regions. Likewise, the liquid impermeable member 112 comprises: a leakage preventing wall-forming portion 112A of a length L11; a flap 112B of a length L12, which includes a front edge 112a; and a flap 112C of a length L13, which includes a rear edge 112b. The flap 112B extends over a part of the front end region; the flap 112C extends over a part of the rear end region; and the leakage preventing wall-forming portion 112A extends over the whole of the intermediate region and the remaining parts of the front and rear end regions.

As in the first embodiment, the individual liquid impermeable members 111 and 112 are formed of an elongated, strip-shaped liquid impermeable sheet 20, which is folded in two and then trimmed. In the flaps 111B and 111C, the liquid impermeable member 111 has free edges 111e and 111f at the fold line. In the leakage preventing wall-forming portion 111A, on the other hand, the liquid impermeable member 111 has a free edge 111d along which the two-ply liquid impermeable sheet 20 is cut. The liquid impermeable member 111 is fixed on the body surface of the main body portion 1 through an adhesive or by heat sealing to form a fixed portion 113 extending longitudinally of the main body portion 1 and having a width W1.

The liquid impermeable member 112 is of configuration symmetrical to the liquid impermeable member 111, and has free edges 112e, 112f in the flaps 112B, 112C and a free edge 112d in the leakage preventing wall-forming portion 112A. The liquid impermeable member 112 is fixed on the body surface of the main body portion 1 to form a fixed portion 114 having a width W1.

The fixed portion 113 extends over the entire length of the leakage preventing wall-forming portion 111A and reaches the end edge 111b of the flap 111C; the fixed portion 114 extends over the entire length of the leakage preventing wall-forming portion 112A and reaches the end edge 112b of the flap 112C. In the leakage preventing wall-forming portion 111A and the flap 111C, therefore, the liquid impermeable member 111 has a free portion (non-fixed portion), which extends from a longitudinally extending starting edge 113a (which is the inner boundary of the fixed portion 113) to the free edges 111d and 111f and can be turned toward the right side edge 1c away from the body surface of the main body portion 1. In the leakage preventing wall-forming portion 112A and the flap 112C, similarly, the liquid impermeable member 112 has a free portion (non-fixed portion), which extends from a longitudinally extending starting edge 114a (which is the inner boundary of the fixed portion 114) to the free edges 112d and 112f and can be turned toward the left side edge 1d away from the body surface of the main body portion 1. The starting edges 113a and 114a are inwardly spaced apart from the right and left side edges 4c and 4d of the absorbent layer 4, respectively, to extend over the absorbent layer 4.

On the other hand, the flaps 111B and 112B provided in the front end regions are not fixed at all on the body surface of the main body portion 1 so that the flaps 111B and 112B can be free.

Over the entire length of the leakage preventing wall-forming portions 111A and 112A, the elastic members 15 and 15 are disposed in and fixed to the two-ply liquid impermeable sheets 20. The individual elastic members 15 are joined to the liquid impermeable sheet 20 while being longitudinally stretched. As a result, in the leakage preventing wall-forming portions 111A and 112A, an elastic contractive force acts on the free portions (non-fixed portions) to reduce their longitudinal dimension.

In the leakage preventing wall-forming portions 111A and 112A, as shown in Fig. 5, the free portions (non-fixed portions) have a transverse dimension (width) W2. That is, the transverse dimension from the starting edges 113a, 114a to the free edges 111d, 112d is indicated by W2. In the flaps 111B, 112B and the flaps 111C, 112C, on the other hand, the free portions (non-fixed portions) have a transverse dimension (width) W3. That is, the transverse dimension from the starting edges 113a, 114a to the free edges 111e, 111f and the free edges 112e, 112f is indicated by W3. Here, the width W3 is set sufficiently larger than the width W2.

In the front flap 111B and the rear flap 111C, the pressure sensitive adhesive layers 23 and 23 as means for detachably fixing are provided on the surfaces appearing in Fig. 5. In the flaps 112B and 112C, similarly, the pressure sensitive adhesive layers 23 and 23 as means for detachably fixing are provided on the surfaces appearing in Fig. 5. Here, it is also possible to use Hook-and-Loop fastener having a large number of small hooks for mechanically engaging with an outer side of an undergarment, as fixing means, in place of the individual pressure sensitive adhesive layers 23.

In the second embodiment, too, the region positioned between the starting edges 113a of the liquid impermeable member 111 and the starting edge 114a of the liquid impermeable member 112 and having the absorbent layer 4 therein is the liquid-receiving region 21.

Next, how to use the sanitary napkin 100 will be described.

As shown in Fig. 5, in a state immediately before wearing the sanitary napkin 100, the liquid impermeable members 111 and 112 are laid on the body surface of the main body portion 1, at the liquid-receiving region 25. At this time, the free edges 111d, 111e and 111f and the free edges 112d, 112e and 112f are positioned closer to the centerline O - O than to the starting edges 113a and 114a. Here, on the body surface of the main body portion 1, the flaps 111B and 112B may overlap with each other and/or the flaps 111C and 112C may overlap with each other.

As shown in Fig. 7, the sanitary napkin 100 is to be used while being attached to an inner side of a crotch portion 30a of an undergarment 30. At this time, the main body portion 1 is put on a central portion of the crotch portion 30a, and the main body portion 1 is fixed on the crotch portion 30a by adhering the pressure sensitive adhesive layers 22 and 22 provided on the garment surface of the main body portion 1 to the inner side of the crotch portion 30a.

Then, the liquid impermeable member 111 is turned toward the side edge 1c about the starting edge 113a, and the liquid impermeable member 112 is turned toward the side edge 1d about the starting edge 114a. As shown in Fig. 7, then, the flaps 111B and 112B are folded back, beyond the side edges of the crotch portion 30a, against an outer side of the undergarment 30 in an wrapping manner, and thereafter, the flaps 111B and 112B are fixed on the outer side of the undergarment 30 through the pressure sensitive adhesive layers 23 and 23. Here, since the flaps 111B and 112B are not fixed at all to the body surface of the main body portion 1 so as to be free, the flaps 111B and 112B can be easily folded back against the outer side of the crotch portion 30a, so that the operation for fixing the flaps 111B and 112B on the outer side of the undergarment 30 is easy.

Similarly, the flaps 111C and 112C are folded back, beyond the side edges of the crotch portion 30a, against the outer side of the undergarment 30, and thereafter, the flaps 111C and 112C are fixed on the outer side through the pressure sensitive adhesive layers.23 and 23.

When the undergarment 30 to which the sanitary napkin 100 is thus attached is worn, the crotch portion 30a of the undergarment 30 is concavely curved to conform to the shape of the wearer's crotch. As a result, the leakage preventing wall-forming portions 111A and 112A are contracted to reduce the longitudinal dimension due to the elastic contractive force of the elastic members 15 and 15, so that they are raised such that their free edges 111d and 112d are moved away from the body surface of the main body portion 1. Then, the free edges 111d and 112d and portions in the vicinities thereof come into contact mainly with the perineal region in the wearer's crotch.

Here, since all the front flaps 111B, 112B and the rear flaps 111C, 112C are fixed to the crotch portion 30a of the undergarment 30, the relative position between the leakage preventing wall-forming portions 111A and 112A and the side edges of the crotch portion 30a is hardly changed.

When worn by pulling up the undergarment 30, the main body portion 1 of the sanitary napkin 100 disposed on the crotch portion 30a tends to convexly deform such that the portion including the centerline O - O is lifted toward the wearer's body, and as a result, the main body portion 1 tends to deform to reduce its transverse dimension. At this time, since the front and rear flaps of the individual liquid impermeable members 111 and 112 are fixed to the side edges of the crotch portion 30a, the liquid impermeable members 111 and 112 are hardly deformed to approach each other, and therefore,. the leakage preventing wall-forming portions 111A and 112A are hardly displaced from the perineal region on two sides of the liquid discharging part of the wearer's body. Accordingly, the liquid body exudate, i.e., the menstrual blood can be certainly received by the liquid-receiving region 21 between the liquid impermeable members 111 and 112 and hardly flows out transversely beyond the liquid impermeable members 111 and 112.

Moreover, since the liquid impermeable members 111 and 112 are constrained by the side edges of the crotch portion 30a, the leakage preventing wall-forming portions 111A and 112A are hardly irregularly deformed due to a pressure from the wearer's body, so that the leakage preventing wall-forming portions 111A and 112A can be maintained in an inclined position having their free edges 111d and 112d directed slightly transversely and outwardly, to come into contact with the wearer's body in such position. Therefore, occurrence of clearance between the leakage preventing wall-forming portions 111A and 112A and the crotch of the wearer's body can be easily prevented.

When the rear portion of the sanitary napkin is brought into contact with the buttocks of the wearer's body, on the other hand, the central portion of the main body portion 1 tends to deform so as to enter the gluteal fold, but even in such case, the leakage preventing wall-forming portions 111A and 112A are hardly irregularly deformed, so that the leakage preventing wall-forming portions 111A and 112A can be easily maintained to contact the perineal region and its surroundings.

Especially in the second embodiment shown in Figs. 5 to 7, the starting edges 113a and 114a, from which the liquid impermeable members 111 and 112 can be raised, are located above the absorbent layer 4. Therefore, even if a deforming force acts to decrease the transverse dimension of the sanitary napkin, the leakage preventing wall-forming portions 111A and 112A are hardly irregularly deformed, since the liquid impermeable members 111 and 112 are positioned over the absorbent layer 4 having some stiffness.

Thus, since the leakage preventing wall-forming portions 111A and 112A can be easily stabilized with the free edges 111d and 112d directed transversely outwardly, the leakage preventing wall-forming portions 111A and 112A are prevented from falling down toward the centerline O - O, so that the liquid impermeable members 111 and 112 can be prevented from overlying with the liquid-receiving region 21 and the decrease of the liquid-receiving area can be prevented.

It should be noted that preferred ranges of the widths W1, W2 and W3 in the second embodiment are the same as those in the first embodiment.

Here, it is also possible that the width W3 is set at about 10 to 25 mm in at least one of the front and rear flaps of each liquid impermeable member so as to be fixed on the inner side of the crotch portion 30a of the undergarment 30 upon use.

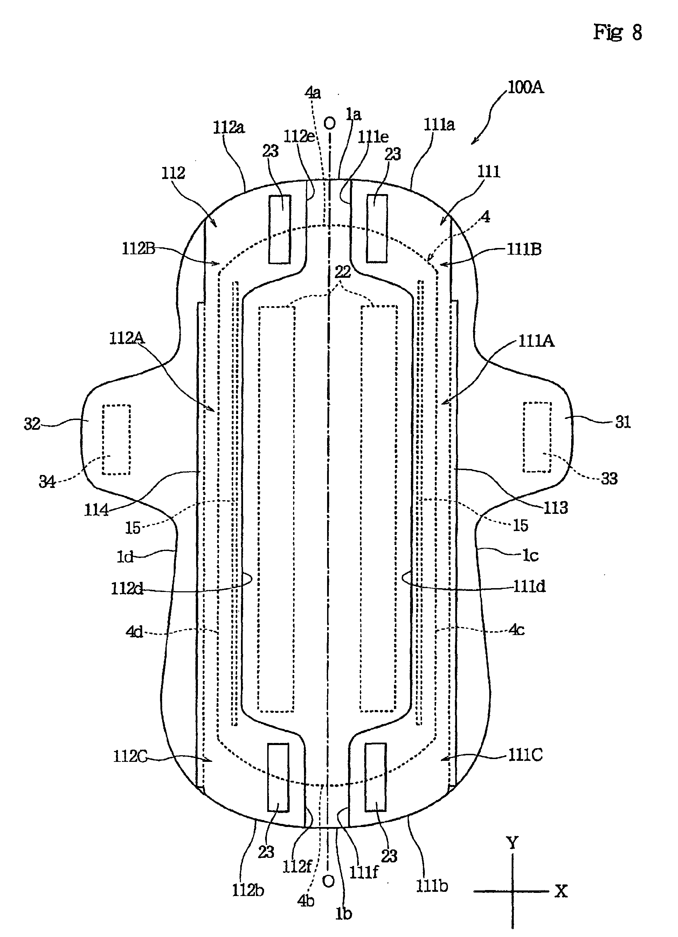

Fig. 8 is a top plan view showing a sanitary napkin 100A as an absorbent article according to a third embodiment of the present invention.

In the third embodiment, the edge shape of a main body portion 1A is different from that of the main body portion 1 shown in Figs. 1 to 7. In the main body portion 1A shown in Fig. 8, part of right and left side edges 1c and 1d are protruded transversely outwardly in a region forwardly offset from a transversely extending centerline of the main body portion 1A, to thereby form wing portions 31 and 32. In the wing portions 31 and 32, pressure sensitive adhesive layers 33 and 34 as detachably fixing means are provided on the exterior surface of the backsheet 3.

The wing portions 31 and 32 are formed by joining the backsheet 3 and the topsheet 2 through a hot-melt adhesive. However, it is also possible that the fixed portions of the liquid impermeable members 111 and 112 are extended to the wing portions 31 and 32 so that the backsheet 3 and the liquid impermeable sheets 20 can be joined through a hot-melt adhesive in the wing portions 31 and 32.

In the third embodiment shown in Fig. 8, moreover, the fixed portion 113, where the liquid impermeable member 111 and the main body portion 1A are fixed, is positioned between the right side edge 4c of the absorbent layer 4 and the right side edge 1c of the main body portion 1A. On the other hand, the fixed portion 114, where the liquid impermeable member 112 and the main body portion 1A are fixed, is positioned between the left side edge 4d of the absorbent layer 4 and the left side edge 1d of the main body portion 1A.

Here, since the shapes and structures of the individual components are identical to those of the embodiment shown in Figs. 5 to 7, except for the wing portions 31 and 32 and the positions of the fixed portions 113 and 114 where the liquid impermeable members 111 and 112 are fixed, the detailed description thereof will be omitted by designating them by the common reference numerals.

When the sanitary napkin of the third embodiment is to be attached to the inner side of the crotch portion 30a of the undergarment 30, the pressure sensitive adhesive layers 22 and 22 disposed on the opposite side of the liquid-receiving region 21 are fixed on the inner side of the crotch portion 30a, and the wing portions 31 and 32 are deformed to be wrapped around the side edges of the crotch portion 30a, and then, the wing portions 31 and 32 are fixed on the outer side of the undergarment 30 through the pressure sensitive adhesive layers 33 and 34.

Moreover, the front flaps 111B, 112B and the rear flaps 111C, 112C are deformed to be wrapped around the side edges of the crotch portion 30a and fixed on the outer side of the undergarment 30, as shown in Fig. 7.

In the third embodiment, since the main body portion 1A can be certainly fixed on the inner side of the crotch portion 30a, the side edges 1c and 1d of the main body portion 1A are hardly deformed to approach each other during wear, so that it becomes possible to prevent the leakage preventing wall-forming portions 111A and 112A from deforming to approach the center more efficiently.

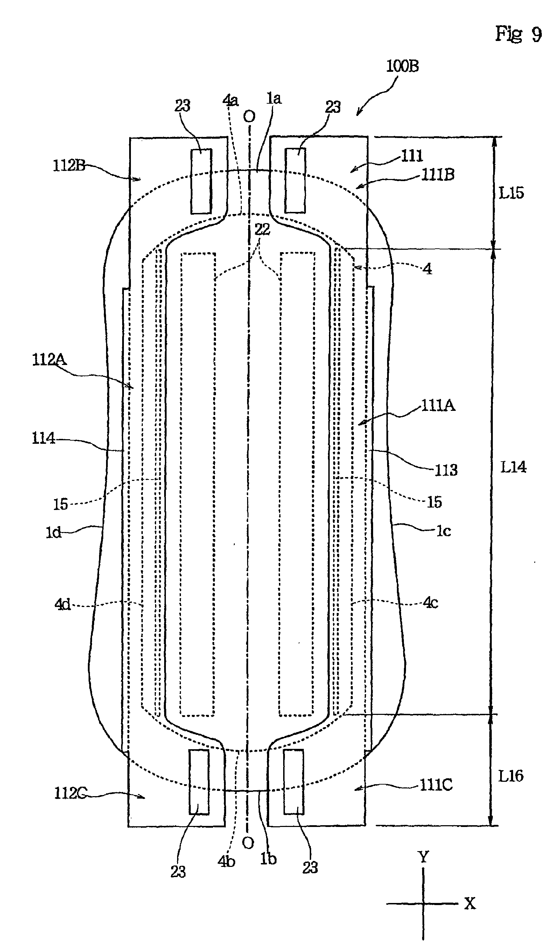

Fig. 9 is a top plan view of a sanitary napkin 100B as an absorbent article according to a fourth embodiment of the present invention. In this embodiment, the shape and structure of the main body portion 1 are identical to those of the embodiments shown in Figs. 1 to 7. Moreover, the shape and structure of the liquid impermeable members 111 and 112 are substantially identical to those shown in Figs. 5 to 7.

In the fourth embodiment shown in Fig. 9, however, the leakage preventing wall-forming portions 111A and 112A are made longer to have a longitudinal dimension L14, the flaps 111B and 112B provided in the front end regions are extended forwardly beyond the front edge 1a of the main body portion 1 to have a longitudinal dimension L15, and the flaps 111C and 112C provided in the rear end regions are extended rearwardly beyond the end edge 1b of the main body portion 1 to have a longitudinal dimension L16. All the flaps 111B, 112B and the flaps 111C, 112C are in a free state as a whole, without joining to the main body portion 1.

In the fourth embodiment, the flaps 111B, 112B and adjacent parts of the leakage preventing wall-forming portions 111A, 112A are not fixed to the main body portion 1. In addition, most part of the flaps 111C, 112C are not fixed to the main body portion 1. Therefore, when the flaps 111B, 112B and the flaps 111C, 112C are transversely turned, they can be extended transversely farther beyond the right and left side edges 1c and 1d. Therefore, the flaps 111B, 112B and the flaps 111C, 112C can be easily folded back against the outer side of the crotch portion 30a of the undergarment 30.

In Fig. 9, it should be noted that the fixed portion 113 of the liquid impermeable member 111 and the fixed portion 114 of the liquid impermeable member 112 are positioned outside the right and left side edges 4c and 4d of the absorbent layer 4 but inside the right and left side edges 1c and 1d of the main body portion 1. The shapes and structures of the other portions are identical to those shown in Figs. 5 to 7, and the detailed description thereof will be omitted by designating them by the common reference numerals.

It is to be understood that the sanitary napkins 100, 100A and 100B, in which the individual liquid impermeable members 111 and 112 are formed with the front and rear flaps as shown in Figs. 5 to 9, may also be used with the flaps fixed on the garment surface of the main body portion 1 or 1A. The flaps may be fixed by a wearer herself upon use or may have been already fixed undetachably from the garment surface.

In the former case, assembly of the sanitary napkin may be completed such that the individual flaps 111B, 112B, 111C and 112C are folded back against the backsheet 3 of the main body portion 1 or 1A, and then, firmly fixed on the exterior surface of the backsheet 3 through a hot-melt type adhesive or by heat sealing so as not to move away therefrom.

In the latter case, the individual flaps 111B, 112B, 111C and 112C may be folded back against the backsheet 3 of the main body portion 1 or 1A by a wearer herself before use, followed by fixing the flaps onto the backsheet 3 using detachably fixing means such as the pressure sensitive adhesive layers 23. The sanitary napkin thus completed is then put on the inner side of the crotch portion 30a of the undergarment 30 for use.

In the case where the individual flaps 111B, 112B, 111C and 112C are fixed on the exterior surface of the backsheet 3 of the main body portion upon use, the leakage preventing walls formed of the liquid impermeable members 111 and 112 are hardly affected by the deformation of the body surface of the main body portion such as undulation, similar to the effect of the first embodiment.

Conversely, it is also possible to use the sanitary napkin 10 of the first embodiment shown in Figs. 2 to 4 as in Fig. 7.

More specifically, the liquid impermeable members 11 and 12 may be transversely outwardly turned after the sanitary napkin 10 of the first embodiment is put on the inner side of the crotch portion 30a of the undergarment 30. Subsequently, the flaps 11B and 12B are deformed to be wrapped around the side edges of the crotch portion 30a, and then fixed on the outer side of the undergarment 30 through the detachably fixing means such as the pressure sensitive adhesive layers provided on the flaps. In this case, even when the sanitary napkin 10 is deformed to reduce its transverse dimension, the leakage preventing walls formed of the liquid impermeable members 11 and 12 are constrained by the undergarment 30 and hardly deformed, similar to the effect of the second to fourth embodiments.

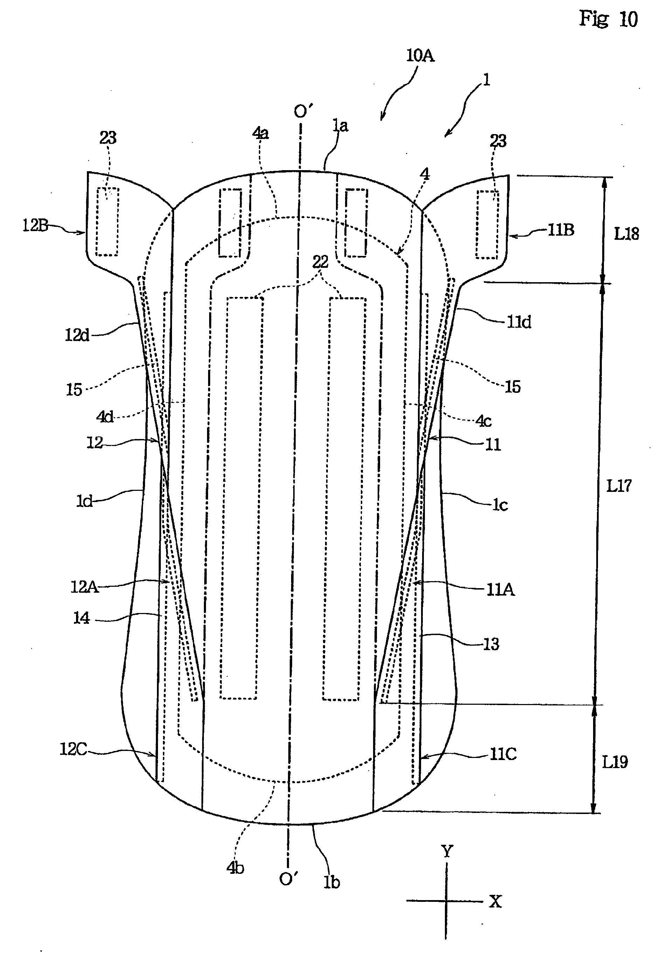

Fig. 10 shows a sanitary napkin 10A according to a fifth embodiment of the present invention, which is more suitable for such case where the flaps are formed only in the front end regions and fixed on the outer side of the undergarment 30 during use.

In the fifth embodiment, the shape and structure of the main body portion are identical to those of the first embodiment shown in Figs. 1 to 4. In addition, the structure of the liquid impermeable members is similar to that of the first embodiment shown in Figs. 1 to 4. Therefore, the detailed description of the portions having the same structure will be omitted by designating them by the common reference numerals.

In the fifth embodiment, the liquid impermeable members 11 and 12 are fixed on the main body portion 1 to form the fixed portion 13 and 14 which extend from the leakage preventing wall-forming portions 11A and 12A of a length L17 to the rear end portions 11C and 12C of a length L19. In the rear end portions 11C and 12C, moreover, the liquid impermeable members 11 and 12 are fixed on the body surface of the main body portion 1 while being laid flat as a whole. In the flaps 11B and 12B of a length L8, on the other hand, the liquid impermeable members 11 and 12 are not fixed at all on the body surface of the main body portion 1 so as to be free. These flaps are provided with the pressure sensitive adhesive layers 23 and 23 as the detachably fixing means.

When the sanitary napkin 10A of the fifth embodiment is to be worn, after the pressure sensitive adhesive layers 22 and 22 provided on the garment surface of the main body portion 1 are adhered to the inner side of the crotch portion 30a of the undergarment 30, the flaps 11B and 12B are transversely outwardly turned, folded back at the side edges of the crotch portion 30a of the undergarment 30, and then fixed on the outer side of the undergarment 30.

Since the flaps 11B and 12B of the fifth embodiment are not fixed on the body surface of the main body portion 1 so as to be free as a whole, as shown in Fig. 10, they can be easily folded back against the outer side of the undergarment 30. It is also possible to further increase the length L18 of the flaps 11B and 12B so that the flaps 11B and 12B can be extended forwardly beyond the front edge 1a of the main body portion 1. Such construction further facilitates folding of the flaps against the undergarment 30.

It is to be understood that the present invention can be variously embodied. For example, in the individual embodiments, the liquid impermeable sheets 20 and 20 forming the liquid impermeable members 11 and 12 (111 and 112) may be extended to the right side edge 1c and the left side edge 1d, respectively.

The absorbent article according to the present invention should not be understood as limited to the sanitary napkin, but may also include a urine absorbing pad which is to be used while being attached to an inner side of an undergarment or disposable diaper, an auxiliary pad for collecting urine and/or feces, and the like.

Next, preferred materials for forming the individual components of the sanitary napkin will be described.

The topsheet 2 may be formed of nonwoven fabric comprising polypropylene fibers, polypropylene fibers, polyethylene terephthalate fibers, bicomponent fibers thereof, or the like, which are treated to be hydrophilic. The nonwoven fabric may be thermal bonded nonwoven fabric, spunbonded nonwoven fabric, spunlaced nonwoven fabric, or the like. In an alternative, the topsheet 2 may be formed of nonwoven fabric in which the synthetic fibers (which may or may not be treated to be hydrophilic) are blended with 5 to 30% by weight of hydrophilic natural fibers or regenerated fibers such as acetate rayon, viscose rayon, cotton or pulp fibers. In another alternative, an apertured resin sheet may also be employed.

The backsheet 3 may be formed of liquid impermeable resin film, spunbonded or spunlaced nonwoven fabric treated to be water repellent, or the like.

The absorbent layer 4 may be formed of comminuted pulp, mixture of comminuted pulp and superabsorbent polymer, or the like, which is wrapped in an absorbent sheet such as tissue paper.

The elastic member 15 may be formed of natural rubber, synthetic rubber, polyurethane, styrene-butadiene copolymer, or the like. It may take the form of yarn, filament, film, strip (belt), or the like. In an alternative, materials cut from stretch nonwoven fabric such as elastic spunbonded or meltblown nonwoven fabric may also be employed.

The liquid impermeable sheet 20 for forming the liquid impermeable members 11, 12, 111 and 112 may be formed of spunbonded or spunlaced nonwoven fabric treated to be water repellent, or the like.

In the absorbent article of the present invention, as has been described hereinabove, at least one end region of each liquid impermeable member is formed with a flap to be wrapped around corresponding one of side edges of a main body portion. Therefore, even if the body surface of the main body portion is irregularly deformed, irregular falling or deformation of leakage preventing walls formed of the liquid impermeable members due to such surface deformation is hardly caused, so that the standing position of the leakage preventing walls can be easily stabilized. Accordingly, the effect of preventing lateral leakage due to the leakage preventing walls can be sufficiently exhibited.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. An absorbent article comprising:
a main body portion including a liquid permeable topsheet on a body surface of the main body portion, a backsheet on a garment surface of the main body portion and an absorbent layer disposed between the topsheet and the backsheet; and
a pair of liquid impermeable members spaced apart from each other transversely of the main body portion and extending longitudinally of the main body portion, each liquid impermeable member having longitudinally opposed front and rear end regions and an intermediate region between the front and rear end regions, wherein
the intermediate region of each liquid impermeable member has a longitudinally extending fixed portion, which is fixed on the body surface of the main body portion, and a non-fixed portion for forming a leakage preventing wall rising from the body surface of the main body portion, which is not fixed on the main body portion and on which an elastic contractive force acts so as to reduce a longitudinal dimension thereof, and
at least one of the front and rear end regions of each liquid impermeable member has a flap which is allowed to be folded back, beyond corresponding one of transversely opposed side edges of the main body portion, against the garment surface of the main body portion.

2. The absorbent article as set forth in claim 1, wherein the flap is folded back against the garment surface of the main body portion and fixed on the backsheet.

3. The absorbent article as set forth in claim 1, wherein at least one of the flap and the backsheet is provided with means for detachably fixing the flap on the backsheet when the flap is folded back against the garment surface of the main body portion.

4. The absorbent article as set forth in claim 1, wherein the flap is provided with means for detachably fixing the flap on an outer side of an undergarment when the absorbent article is put on an inner side of the undergarment and the flap is folded back to come into contact with the outer side of the undergarment.

5. The absorbent article as set forth in claim 1, wherein only one of the front and rear end regions of each liquid impermeable member is formed with the flap, and the other end region is fixed on the body surface of the main body portion as a whole so as not to be movable away from the body surface of the main body portion.

6. The absorbent article as set forth in claim 1, wherein both the front and rear end regions of each liquid impermeable member are formed with flaps.

7. The absorbent article as set forth in claim 1, wherein the flap is not fixed on the body surface of the main body portion so as to be movable away from the body surface of the main body portion as a whole.

8. The absorbent article as set forth in claim 1, wherein the flap is extended outwardly beyond corresponding one of longitudinally opposed front and rear edges of the main body portion.

9. The absorbent article as set forth in claim 1, wherein a boundary between the fixed portion and the non-fixed portion of the intermediate region is positioned above the absorbent layer.

10. The absorbent article as set forth in claim 1, wherein a free edge of the liquid impermeable member is located closer to a longitudinally extending centerline of the main body portion than the fixed portion when the liquid impermeable member is not moved away from but remains laid on the body surface of the main body portion.
